# EUROPEAN PATENT SPECIFICATION

(11) **EP 1 252 521 B1**
(45) Date of publication and mention of the grant of the patent: **29.12.2004**
(21) Application number: 01911576.5
(22) Date of filing: 02.02.2001
(51) Int. Cl.: G01N 33/68, G01N 33/50, G01N 33/543, G01N 33/569

(54) **PROCESS FOR THE IDENTIFICATION OF IGG-PRODUCING CELLS**
VERFAHREN ZUR IDENTIFIZIERUNG VON IGG-PRODUZIERENDEN ZELLEN
PROCEDE DESTINE A DISCRIMINER DES CELLULES PRODUCTRICES D'IGG DE CELLULES NON PRODUCTRICES D'IGG

(30) Priority: 03.02.2000 GB 0002539
(43) Date of publication of application: 30.10.2002
(73) Proprietor: Lonza Group AG, 4052 Basel (CH)
(72) Inventor: BIRCH, John, High Wycombe, Buckinghamshire HP11 1PH (GB); METCALFE, Hilary, Burbage, Wiltshire SN8 3TY (GB); KNIGHT, Adrian, Middlesbrough, TS7 8PU (GB); VERNON, Alison, Reading, Berkshire RG6 3BA (GB)
(86) International application number: PCT/EP2001/001100
(87) International publication number: WO 2001/057527

(56) References cited:
- WO-A-89/10566
- WEAVER J C ET AL: "Gel microdrop technology for rapid isolation of rare and high producer cells" NATURE MEDICINE, NATURE PUBLISHING, CO, US, vol. 3, no. 5, May 1997 (1997-05), pages 583-585, XP002116070 ISSN: 1078-8956
- KENNEY JOHN S ET AL: "Production of monoclonal antibodies using a secretion capture report web." BIO-TECHNOLOGY (NEW YORK), vol. 13, no. 8, 1995, pages 787-790, XP001015788 ISSN: 0733-222X cited in the application
- GRAY F ET AL: "Secretion capture and report web: use of affinity derivatized agarose microdroplets for the selection of hybridoma cells" JOURNAL OF IMMUNOLOGICAL METHODS, ELSEVIER SCIENCE PUBLISHERS B.V.,AMSTERDAM, NL, vol. 182, no. 2, 9 June 1995 (1995-06-09), pages 155-163, XP004021164 ISSN: 0022-1759 cited in the application
- AL-RUBEAI M ET AL: "Flow cytometry in animal cell culture" BIO/TECHNOLOGY, NATURE PUBLISHING CO. NEW YORK, US, vol. 11, May 1993 (1993-05), pages 572-579, XP002116068 ISSN: 0733-222X
- MEININGER ET AL BIOCHEMISTRY, AMERICAN CHEMICAL SOCIETY vol. 39, no. 1, 11 January 2000, pages 26 - 36
- ERNTELL ET AL MOLECULAR IMMUNOLOGY vol. 25, no. 2, 1988, pages 121 - 126

## Description

The present invention relates to a novel process for the identification of IgG-producing cells from non-IgG-producing cells by discriminating fluorescently labelled encapsulated IgG-producing and non-IgG-producing cells using flow cytometry.

Immunoglobulin G (IgG) is increasingly used for diagnostic and therapeutic purposes in human medicine (Scheinberg, D. A. and Chapman, P. B., in Birch, J. R.and Lennox, E. S., eds., 1995, Wiley, 45-105).

Identification methods which uses encapsulation of cells in order to allow for discriminating of IgG-producing cells from non-IgG-producing cells are well known (Gray, F. et al., 1995, J. Imm. Meth., 182, 155-163; Kenney, J. S. et al., 1995, Bio/Tech 13, 787-790) and are as well commercially available e. g. from One Cell Systems Inc. (Cambridge, MA). However, the methods described have some disadvantages in that they do not give sufficient resolution for the identification of high producing cell lines.

WO89/10566 discloses a process for forming and using gel microdroplets (GMD), wherein Protein A can be added as labelling molecule.

Meininger et al. (2000, Vol. 39, no. 1, pages 26-36) describe the capability of Protein A and Protein G to bind to the Fc portion of immunoglobulin.

The object of the present invention is to provide an improved process which has a greater resolution which allows to identify high levels of high producing cell lines.

This object has been achieved with a novel process according to claim 1.
According to the invention IgG-producing cells are identified from non-IgG-producing cells whereby
a) the IgG-producing cells and the non-IgG-producing cells are encapsulated in a matrix which contains a capture reagent for the IgG produced
b) said matrix is incubated for a period of time during which IgG can be produced
c) said produced IgG is fluorescently labelled
d) and fluorescently labelled encapsulated IgG-producing cells and non-IgG-producing cells are discriminated using flow-cytometry
whereby the capture reagent is Protein A and/or Protein G.

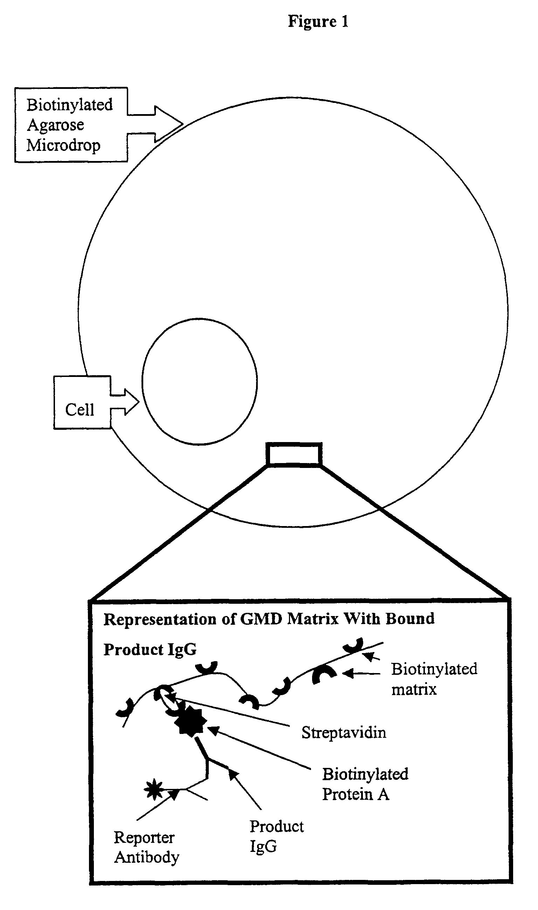

**Figure 1** shows schematic for the capture of IgG produced by an encapsulated cell.
**Figure 2** shows capture of IgG by using Protein A in comparison to antibody as capture reagent
**Figure 3** shows working concentrations of Protein A

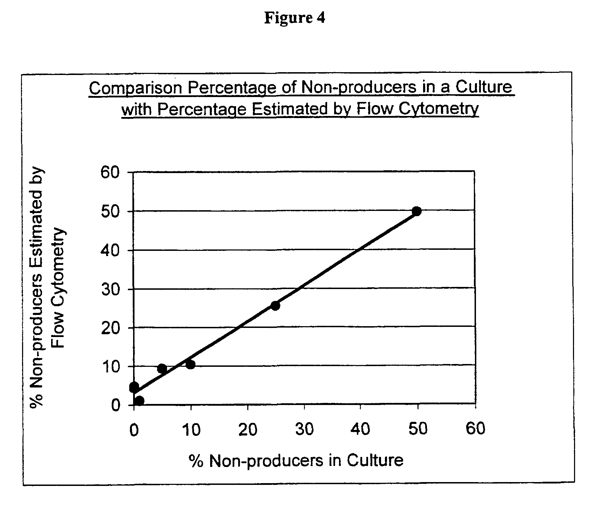

**Figure 4** shows comparison percentage of GS-NS0 non-producers in a culture with percentage estimated by flow cytometry

### Cells

Depending on the efficiency of expression and secretion of IgG, the cells employed in the present invention are defined as IgG-producing cells or non-IgG producing cells. Non-IgG producing cells might express and secrete low amounts of IgG up to about 0.1 to 0.2 µg/ml or might express and secrete no IgG at all. IgG-producing cells might express and secrete IgG at levels above 0.2 µg/ml. Preferred cells which can be discriminated into IgG-producing cells and non-IgG-producing cells are mammalian cells.
Every mammalian cell can be used for the present invention. Examples for mammalian cells which can be used are plastocytoma, myeloma, hybridoma, Chinese Hamster Ovary (CHO), fibrosarcoma or lymphoblastoid cells of murine or human origin. Preferred mammalian cells used in the present invention are murine myeloma cells. Examples for murine myeloma cells are NS0 and NS0 transfected with Glutamine-Synthetase (GS-NSO). Particular useful are GS-NS0.

### IgG

IgG which can be produced by the cells used in the present invention might be human, swine, guinea pig, bovine, goat, sheep, monkey, horse, mouse, rat or hamster IgG. Humanised and chimeric IgG might be produced as well. Preferably, human IgG is produced.
Furthermore the cells might as well produce fragments of IgG, like Fc-, Fab-, (Fab)₂-fragments.

### Encapsulation

Encapsulation of the IgG-producing cells and non-IgG producing cells can be performed by using e.g. standard methods as described in WO 82/02561 or according to protocols provided by One Cell Systems Inc.
Usually the cells are added to a liquefied gel containing the encapsulation matrix like the CelBioGel ™ encapsulation matrix commercialized by One Cell Systems Inc.. The encapsulation matrix usually consists of agarose. The matrix might be biotinylated. Particular preferred are biotinylated agarose matrices.
The preferred number of cells which will be added to the liquefied gel is between 10⁵ and 10⁸ cells/ml, particular between 5x10⁵ and 10⁷ cells/ml.

The cells in the liquefied encapsulation matrix can be further processed to generate gel microdroplets (GMD) containing at least one cell per GMD by using an emulsion matrix which usually consists of oil, preferably of mineral oil, like the CelMix™ emulsion matrix and a microdrop maker like the CellSys 101™ microdrop maker. CelMix™ emulsion matrix and CellSys 101™ microdrop maker are commercialized by One Cell Systems Inc..

The capture reagent Protein A and/or Protein G can be bound to the matrix before or after GMDs have been generated. Preferably, Protein A and/or Protein G is bound after GMDs have been generated. Binding occurs usually by first incubating the matrix with a linker followed by incubation with Protein A and/or Protein G. In case the matrix is biotinylated, a preferred linker is an avidin, like streptavidin. Particularly used is streptavidin.
Protein A and/or Protein G might be biotinylated in order to be capable to bind to the linker in case the linker is avidin. Preferably, biotinylated Protein A and/or Protein G is used, in particular biotinylated Protein A is used. The usual amount of Protein A and/or Protein G added to the matrix or to the GMDs is between 0.001 and 5 mg/ml, preferably between 0.01 and 0.1 mg/ml.

### Incubation for IgG production

The incubation period during which IgG can be produced depends on the specific IgG-productivity of the the cell as well as on the kind of cell used. In case a murine myloma cell is used the period of time is usually between 1 and 15 minutes, in particular between 3 and 12 minutes.

The incubation temperature depends as well on the specific IgG-productivity of the cell as well as on the kind of cell used. In case a murine myloma cell is used the incubation is carried out at a temperature of between 2 °C and 15 °C, preferably between 2°C and 8 °C.
Usually a control IgG is added during incubation in order to validate the binding of the capture reagent.

### Labelling and selection by flow cytometry

As a fluorescent label anything which can recognize the product IgG and which is conjugated to a fluorescent compound can be used. Usually, antibodies which are conjugated to fluorescent compound and which are specific for the product IgG or antigens which are conjugated to fluorescent compound and which are specific for the product IgG can be used. Preferably , antibodies which are conjugated to fluorescent compound and which are specific for the product IgG are used. Antibodies specific for the product IgG may be Immunoglobulins selected from the Immunoglobulin classes A, D, E, G or M and should be derived from a host cell not related to the cell which express and secrete product IgG. Preferably, the antibody specific for the produced IgG is as well an IgG. In case the cell which express and secrete product IgG is a murine cell, the labelling antibody might be e.g. an IgG derived from goat.

Examples for fluorescent compounds are Phycoerythrin, Fluoresceinisothiocyanate, Texas Red, Rhodamine or Cyanine dyes. As a preferred fluorescent label an IgG which is conjugated to phycoerythrin and which is specific for the product IgG is used. IgGs which contain a fluorescent compound and which are specific for the product IgG are commercially available.
In case antibodies specific for the product IgG are used as label, preferably a blocker is added to the encapsulated cells before labelling in order to prevent additional secreted product from binding as well as to reduce non specific binding. As a preferred blocker serum, particularly foetal calf serum, or a solution of skimmed milk (e.g. Marvel) is used.

The usual flow cytometry conditions in order to discriminate IgG-producing cells from non-IgG-producing cells are chosen according to the protocol provided by One Cell Systems Inc.. Usually empty GMDs and GMDs which contain at least one cell are detected by scatter characteristics displayed on a plot of linear forward scatter (FSc) and log side scatter (SSc). A negative control (no capture reagent) is run first and the FSc and SSc are adjusted until the occupied population appears in approximately the centre of the FSc/SSc plot. The threshold discriminator is adjusted to reduce the appearane of the empty GMDs. The singly occupied sub-population is gated and analysed for fluoresence using a log setting on the detection PMT (photomultiplier tube) appropriate to the fluorochrome. The fluorescence detection PMT is adjusted so that the population lies in the first log decade of the fluoresence histogram. Positive and test samples are then run, generally 10,000 events are collected per sample. Discrimination by flow cytometry can occur analytically or preperatively. Optionally, GMD might be stained before flow cytometry in order to select for viable cells by exposing the cells to a staining compound like propidium iodide.

Detection limits for IgG producing cells in a population of IgG-producing cells and non-IgG-producing cells usually obtained are between 95 and 99.99 %, preferably between 99 and 99.5 % of the total cell number.

A further subject of the present invention is a biotinylated agarose matrix containing biotinylated Protein A and /or Protein G as capture reagent for IgG whereby the biotinylated Protein A and /or Protein G is bound by an avidin linker to the matrix. Preferably, the matrix contains Protein A as capture reagent for IgG and the avidin linker is streptavidin.

### Examples:

### Example 1

### Preparation of cells and matrix

15 ml of CelMix™ 200cs emulsion matrix were equilibrated in a sterile glass scintillation vial to 37°C. 10⁶ viable GS-NS0 cells were harvested by centrifuging at 200 x g for 10 min. The cells were resuspended in 100 µl phosphate buffered saline (PBS). 25 µl Pluronic acid (sterile filtered) were added to 400 µl of liquefied CelBioGel™ and the gel was equilibrated at 37°C for 3 min. The resuspended cells were added to the CelBioGel™ and were mixed slightly and incubated at 37°C for 3 min. The CelBioGel™ cell mixture were added dropwise in a spiral pattern (from bottom to top) to the warmed emulsion matrix oil in the vial using a 1ml pipette tip, avoiding adding air bubbles. This mixture was emulsified using the CellSys 101™ microdrop maker which produced 25 µm microdrops by centrifugatung 2100 rpm for 1 min at RT, 2100 rpm for 1 min in ice bath and 1100 rpm for 10 min in ice bath. The encapsulation mixture was splited into two 15ml conical tubes and the emulsion was carefully overlayed with 5 ml PBS. The solution was spinned for 10 min at 600g, so that the GMD pellet was visible. The oil phase was removed with a pipette, the supematent was aspirated and the pellets were combined from the two tubes and 2 ml PBS was added. 10 ml PBS was aliquoted into a 15 ml conical tube and overlayed with the GMD's. The mixture was spinned for 5min at 400 x g (repeat step if necessary). The supematent was removed and cells were resuspended in appropriate solution.

### A. Streptavidin Bridge

The GMD pellet were resuspended in 2.3 ml cold DMEM medium and a 10 µl aliquot was evaluated for % occupancy. 0.3 ml were saved for "unstained" negative control. 80 µl of 1 mg/ml streptavidin were diluted in 2 ml cold L15 cell culture medium (Biowhittaker) and added to the 2 ml of GMDs and incubated on a rotator at RT for 15 min. The mixture was washed in 10 ml cold PBS (Spinned twice at 400 x g for 5 min).

### B. Biotinylated Capture IgG Binding

The GMDs was resuspended in 2.3 ml L 15 (0.3 ml was saved as "no-capture" negative controls). 80 µl of 1.0 mg/ml biotinylated Protein A capture reagent (Pierce) were diluted in 2 ml cold L15. Diluted capture was added to the 2 ml of GMDs and the solution was incubated on a rotator at RT for 15 min and washed in cold PBS (spinned twice at 400 x g for 5 min) and resuspended in 3.3 ml cold L15 ( 0.3 ml were saved for positive controls).

### C. Secretion Incubation of Encapsulated Cells

GMDs were added to 25 ml cold L15 and equilibrated at 4°C in T75 flask and incubated at 4°C for 10 min. 6 µl of control IgG (Chromepure human IgG) were diluted in 0.8 ml L15. 0.4 ml of the diluted control IgG were added to each control and this solution was incubated on rotator at RT for 15-30 min. At the end of these incubations all samples were washed twice in cold PBS/ 2% foetal bovine serum (FBS). (Spinned at 400 x g for 5 min ). The secretion sample was resuspended in 2 ml and the controls in 1 ml each cold L15 + 5% FBS.

### D. Staining with Detection Antibody

40 µl of 0.5 mg/ml goat anti-human IgG-Anti-f(ab')₂-R-Phycoerythrin (R-PE) detection antibody were diluted in 2 ml cold L15 + 5% FBS for the sample and 4 µl in 2 ml for the controls. 40 µl preparation was added to the GMD's and 1 ml from the 4 µl preparation was added to each of the controls. The mixture was rotated for 15 min at RT (in darkness). It was washed 2 x in 10 ml cold PBS/ 2% FBS. (Spinned at 400 x g for 5 min ) and each sample was resuspended in 2.0 ml of PBS for flow cytometry.

### E. Flow Cytometry of Encapsulated Cells

The instrument used was a Coulter EPICS ELITE cell sorter equipped with a 488nm argon ion laser pre-aligned with Flowcheck fluorospheres. Empty and occupied GMDs are detected by scatter characteristics displayed on a plot of linear forward scatter (FSc) and log side scatter (SSc). The negative control (no capture reagent) was run first and the FSc and SSc are adjusted until the occupied population appears in approximately the centre of the FSc/SSc plot. The threshold discriminator was adjusted to reduce the appearance of the empty GMDs. The singly occupied sub-population was gated and analysed for fluorescence using a log setting on the detection PMT (photomultiplier tube) appropriate to the fluorochrome. The fluorescence detection PMT was adjusted so that the population lies in the first log decade of the fluorescence histogram. Positive and test samples were then run, generally 10,000 events are collected per sample.

### Example 2

### Comparison of capture of product chimeric IgG by using Protein A and antibody as capture reagent, respectively

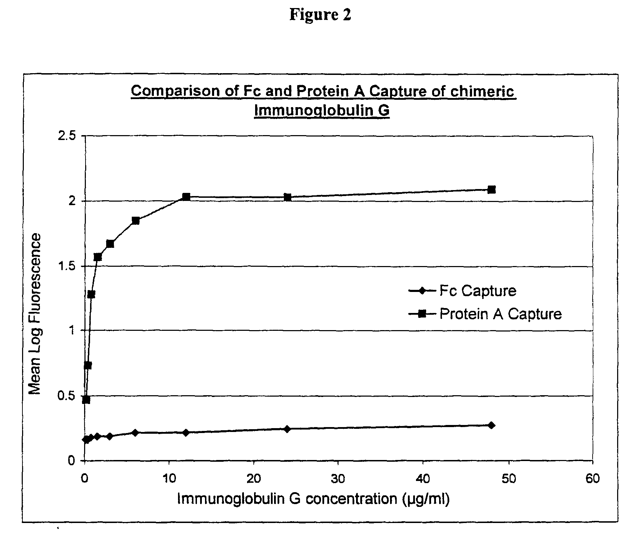

Different dilutions of the product chimeric IgG were exposed to empty GMDs with either biotinylated Protein A capture reagent or biotinylated goat anti-human IgG Fc gamma fragment specific antibody capture reagent. GMDs incorporating Protein A showed much higher fluorescence compared to the antibody as capture reagent at all concentrations of product (see figure 2).

### Example 3

### Determination of working concentrations of Protein A

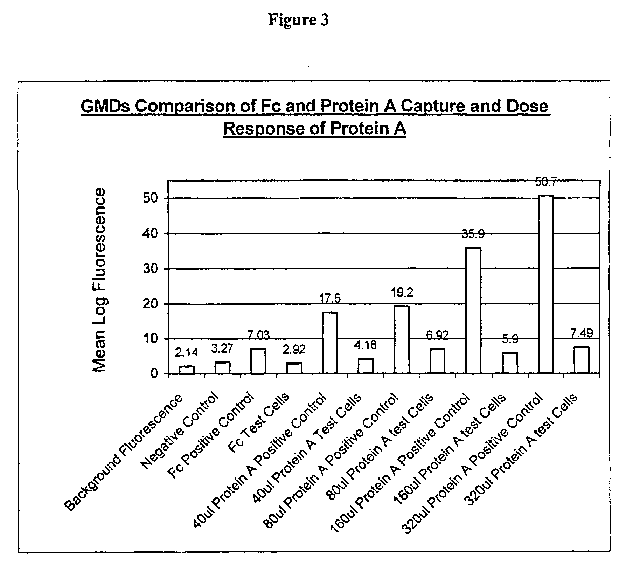

A GS-NS0 cell line was used as encapsulated test cells which produce IgG product. As negative control encapsulated cells without biotinylated protein A was used. As a Fc positive control encapsulated cells producing IgG were spiked with human IgG. A stock solution of 1mg Protein A per ml of PBS was used (see figure 3).

## Claims

1. A process for the identification of IgG-producing cells from non-IgG-producing cells whereby
a) the IgG-producing cells and the non-IgG-producing cells are encapsulated in a matrix which contains a capture reagent for the IgG produced,
b) said matrix is incubated for a period of time during which IgG can be produced
c) said produced IgG is fluorescently labelled
d) and fluorescently labelled encapsulated IgG-producing cells and non-IgG-producing cells are discriminated using flow-cytometry
**characterised in that** the capture reagent is Protein A and/or Protein G.

2. A process of claim 1, wherein the non-IgG-producing cells and the IgG-producing cells are mammalian cells.

3. A process of claim 2, wherein the mammalian cells are murine myeloma cells.

4. A process of any of claim 1 to 3, wherein the IgG produced is a human IgG.

5. A process of any of claim 1 to 4, wherein Protein A and/or Protein G is biotinylated.

6. A process of claim 3, wherein the period of time during which IgG can be produced is between 1 and 15 minutes.

7. A process of claim 3, wherein the incubation is carried out at a temperature of between 2°C and 15°C.

8. A biotinylated agarose matrix containing biotinylated Protein A and /or Protein G as capture reagent for IgG whereby the biotinylated Protein A and /or Protein G is bound by an avidin linker to the matrix.

## Patentansprüche

1. Verfahren zur Identifizierung IgG produzierender Zellen unter nicht-IgG produzierenden Zellen, wodurch
a) die IgG produzierenden Zellen und die nicht-IgG produzierenden Zellen in einer Matrix, die ein Rückhaltereagens für den produzierten Antikörper enthält, eingekapselt sind,
b) besagte Matrix für eine Zeitspanne, während der IgG produziert werden kann, inkubiert wird,
c) besagter produzierter IgG fluoreszenzmarkiert wird,
d) und fluoreszenzmarkierte, eingekapselte IgG-produzierende Zellen und nicht-IgG produzierende Zellen durch Durchflusscytometrie unterschieden werden, **dadurch gekennzeichnet, dass** das Rückhaltereagens Protein A und/oder Protein G ist.

2. Verfahren nach Anspruch 1, **dadurch gekennzeichnet, dass** die nicht IgGproduzierenden Zellen und die IgG produzierenden Zellen Säugerzellen sind.

3. Verfahren nach Anspruch 2, **dadurch gekennzeichnet, dass** die Säugerzellen Maus-Myelomazellen sind.

4. Verfahren nach einem der Ansprüche 1 bis 3, **dadurch gekennzeichnet, dass** der produzierte IgG ein humaner IgG ist.

5. Verfahren nach einem der Ansprüche 1 bis 4, **dadurch gekennzeichnet, dass** das Protein A und/oder Protein G biotinyliert ist.

6. Verfahren nach Anspruch 3, **dadurch gekennzeichnet, dass** die Zeitspanne während der IgG produziert werden kann, zwischen 1 und 15 Minuten beträgt.

7. Verfahren nach Anspruch 3, **dadurch gekennzeichnet, dass** die Inkubation bei einer Temperatur zwischen 2°C und 15°C ausgeführt wird.

8. Eine biotinylierte Agarose-Matrix umfassend biotinyliertes Protein A und/oder Protein G als Rückhaltereagens für IgG wobei das biotinylierte Protein A und/oder Protein G vermittels eines Avidin-Verbindungsstückes an die Matrix gebunden ist.

## Revendications

1. Processus pour l'identification de cellules productrices d'IgG parmi des cellules non productrices d'IgG, moyennant quoi
a) les cellules productrices d'IgG et les cellules non productrices d'IgG sont encapsulées dans une matrice qui contient un réactif de capture des IgG produites,
b) ladite matrice est incubée pendant une période durant laquelle des IgG peuvent être produites
c) lesdites IgG produites sont marquées par fluorescence
d) et les cellules productrices d'IgG ainsi que les cellules non productrices d'IgG, encapsulées et marquées par fluorescence, sont distinguées en utilisant la cytométrie de flux **caractérisé en ce que** le réactif de capture est une protéine A et/ou une protéine G.

2. Processus selon la revendication 1, dans lequel les cellules non productrices d'IgG et les cellules productrices d'IgG sont des cellules de mammifère.

3. Processus selon la revendication 2, dans lequel les cellules de mammifère sont des cellules de myélome murin.

4. Processus selon l'une quelconque des revendications 1 à 3, dans lequel les IgG produites sont des IgG humaines.

5. Processus selon l'une quelconque des revendications 1 à 4, dans lequel la protéine A et/ou la protéine G sont biotinylées.

6. Processus selon la revendication 3, dans lequel la période durant laquelle des IgG peuvent être produites est comprise entre 1 et 15 minutes.

7. Processus selon la revendication 3, dans lequel l'incubation est effectuée à une température comprise entre 2°C et 15°C.

8. Matrice d'agarose biotinylée contenant de la protéine A et/ou de la protéine G biotinylées comme réactifs de capture pour des IgG, moyennant quoi la protéine A et/ou la protéine G biotinylées sont liées à la matrice par un lieur d'avidine.
